# EUROPEAN PATENT SPECIFICATION

(11) **EP 2 465 063 B1**
(45) Date of publication and mention of the grant of the patent: **31.01.2018**
(21) Application number: 10752465.4
(22) Date of filing: 11.08.2010
(51) Int. Cl.: G06F 19/00

(54) **GRAPHICAL INTERFACE FOR ANALYTE METER**
GRAFISCHE SCHNITTSTELLE FÜR EINEN ANALYTMESSER
INTERFACE GRAPHIQUE POUR APPAREIL DE MESURE D'ANALYTE

(30) Priority: 11.08.2009 US 233113 P
(43) Date of publication of application: 20.06.2012
(73) Proprietor: Ascensia Diabetes Care Holdings AG, 4052 Basel (CH)
(72) Inventor: GAONKAR, Kripa, Tarrytown New York 10591 (US); RIPLEY, Paul, M., Nanuet New York 10954 (US); SUN, Hoi-Cheong, Steve, Mount Kisco New York 10549 (US); TELSON, Stan, White Plains New York 10603 (US); WU, Mu, Hopewell Junction New York 12533 (US); DUNNE, Nancy, Sleepy Hollow New York 10591 (US)
(74) Representative: Cohausz & Florack
(86) International application number: PCT/US2010/045175
(87) International publication number: WO 2011/019820

(56) References cited:
- EP-A2- 1 457 913
- US-A1- 2003 176 183
- US-A1- 2005 001 179

## Description

### CROSS-REFERENCE TO RELATED APPLICATIONS

This application claims priority to U.S. Provisional Patent Application No. 61/233,113, filed August 11, 2009, the contents of which are incorporated entirely herein by reference.

### FIELD OF THE INVENTION

The present invention relates generally to methods and systems for presenting information to a user of a diagnostic system. More specifically, the methods and systems according to aspects of the present invention provide a graphical user interface for a diagnostic system. Additionally, the graphical user interface provides information for operating the diagnostic system.

### BACKGROUND OF THE INVENTION

The quantitative determination of analytes in body fluids is of great importance in the diagnoses and maintenance of certain physiological abnormalities. For example, lactate, cholesterol and bilirubin are monitored in certain individuals. In particular, it is important that individuals with diabetes frequently check the glucose level in their body fluids to regulate the glucose intake in their diets. The results of such tests can be used to determine what, if any, insulin or other medication needs to be administered.

Diagnostic systems, such as blood-glucose systems, may employ a meter or instrument to calculate the concentration of an analyte in a sample of body fluid. In some types of diagnostic systems, test sensors are used to test a sample of blood. A test sensor contains biosensing or reagent material that reacts with the analyte, e.g., blood glucose, in the sample. For example, the testing end of the sensor may be placed into contact with the fluid being tested (e.g., blood) that has accumulated on a person's finger after the finger has been pricked. A sufficient amount of fluid to be tested may be drawn from the testing end by capillary action to the reagent material in the sensor. The meter receives the test sensor and applies optical or electrochemical testing methods to by measure an output, such as current or color, from the reaction between the analyte and the reagent in the test sensor. Diagnostic systems typically employ a graphical user interface to display the results of the testing to the user. The graphical user interface may also be employed to display instructions to the user.

Diagnostic systems require the user to complete several steps during the testing procedure. The accuracy of such testing methods, however, depend on the manner in which the user completes the steps. Furthermore, document US 2003/0176183 A1 discloses a module acting as a meter and configured to receive a test strip. This meter includes a glucose measurement circuit and can be connected to a computer. In addition, document EP 1 457 913 A2 relates to a method of managing data for a plurality of analyte test instruments connected to a data communication network comprising the steps of: detecting via host computer the connection of each instrument to the data communication network; uploading data received from each instrument to the host computer; processing the upload data on the host computer for operational review; and downloading configuration data from the host computer to each test instrument, the downloaded data comprising instrument specific set up and control data.

### SUMMARY OF THE INVENTION

In view of the foregoing, it would be desirable to have systems and methods that provide a diagnostic system with a graphical user interface that provides users with clear and easy-to-follow instructions for conducting the testing procedure of diagnostic systems to produce accurate results. Moreover, it would be desirable to have a graphical user interface that provides users with instructions on how to operate the diagnostic systems when an error or exceptional condition arises.The objects are solved according to the present invention by a system according to claim 1.

Accordingly, diagnostic systems according to aspects of the present invention include a meter that is configured to receive a test sensor during a testing procedure. The diagnostic systems also include a computing device coupled to the meter. The test sensor receives a fluid sample during the testing procedure. The meter includes a measurement system that determines a measurement of a concentration of an analyte in the fluid sample. The computing device receives and processes the measurement from the meter. In particular, the computing device has enhanced processing and presentation capabilities that provide visual and/or audio instructions on how to operate the diagnostic systems, especially when an error or exceptional condition arises.

Diagnostic systems according to aspects of the present invention employ a graphical user interface (GUI), or display, that provides clear and easy-to-follow instructions for conducting the testing procedure. For example, a processing device in a diagnostic system executes software that is stored on computer-readable media to present illustrative graphics, textual information, and/or audio on a corresponding user interface for each step during the testing procedure. As such, the user receives clear step-by-step instructions to minimize the chance of user error during the testing procedure. In some embodiments, the software may enhance the presentation of instructions by employing animation.

In some embodiments, the GUI also presents illustrative graphics, textual information, and/or audio that guide the user through appropriate steps when an error or exceptional condition occurs during the testing procedure. For example, because the result of the chemical reaction between the analyte and a reagent on the test sensor may vary at different temperatures, the accuracy of the testing procedure may be affected by the temperature of the test sensor. Although the actual measurement may be corrected based on the actual test sensor temperature taken right before the reaction begins, in some cases, the accuracy of the testing procedure is improved by replacing the test sensor with one that has a temperature within a preferred range. Thus, in some embodiments, the GUI presents illustrative graphics, textual information, and/or audio that instruct the user to replace the test sensor when the diagnostic system senses that the test sensor temperature is outside a preferred range.

Although the meter may include the processing device, the software, and the GUI for presenting the illustrative graphics, textual information, and/or audio, it is understood that diagnostic systems according to the aspects of the present invention employ a variety of architectures. For example, a diagnostic system employs a meter in combination with an external device, such as a conventional personal computer, a personal data assistant (PDA), or smart phone. As such, the software is loaded on the external device to allow a processor of the external device to execute the software and to present illustrative graphics, textual information, and/or audio on a user interface of the external device. Indeed, in some embodiments, the software is a part of an data management system that is executed on the external device to manage, analyze, and present test results that have been stored by the meter. Is such embodiments, the data management system takes advantage of greater processing and display capabilities to provide enhanced functionality, which may not be otherwise possible with the processor and user interface on a meter.

While it may be advantageous to show the illustrative graphics, textual information, and/or audio during the actual testing procedure, it is understood that the illustrative graphics and/or textual information may be shown separately as a tutorial.

Still other aspects, features, and advantages of the present invention are readily apparent from the following detailed description, by illustrating a number of exemplary embodiments and implementations, including the best mode contemplated for carrying out the present invention. The present invention is also capable of other and different embodiments, and its several details can be modified in various respects, all without departing from the spirit and scope of the present invention. Accordingly, the drawings and descriptions are to be regarded as illustrative in nature, and not as restrictive. The invention is to cover all modifications, equivalents, and alternatives falling within the spirit and scope of the invention.

### BRIEF DESCRIPTION OF THE DRAWINGS

FIG. 1 illustrates an embodiment of a diagnostic system including meter and a computing device according to aspects of the present invention.
FIG. 2A illustrates a connection between a meter and a computing device.
FIG. 2B illustrates a connection between a meter and a computing device that provides secondary isolation/protection from the power source of the computing device, according to aspects of the present invention.
FIG. 2C illustrates another connection between a meter and a computing device that provides secondary isolation/protection from the power source of the computing device.
FIG. 3 illustrates a miniature blood glucose meter according to aspects of the present invention.
FIG. 4 illustrates the miniature blood glucose meter coupled to a smart phone according to aspects of the present invention.
FIGS. 5A-E illustrates example graphical and textual information that may be displayed by a diagnostic system according to aspects of the present invention.
FIG. 6 illustrates an alternative embodiment of a meter according to aspects of the present invention.
FIG. 7 illustrates another alternative embodiment of a meter according to aspects of the present invention.
FIG. 8 illustrates yet another alternative embodiment of a meter according to aspects of the present invention.

### DESCRIPTION OF ILLUSTRATED EMBODIMENTS

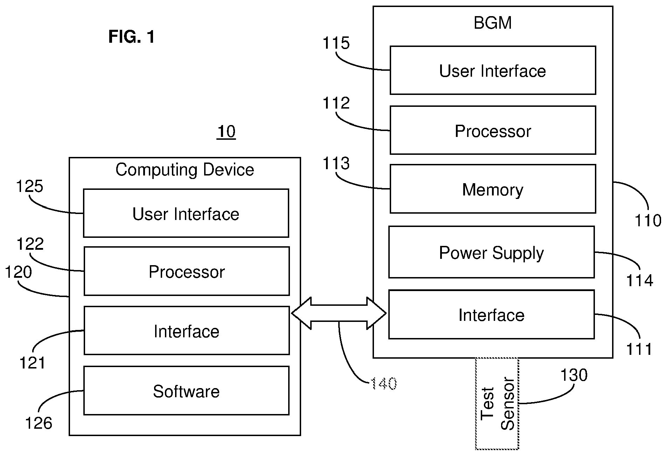

FIG. 1 provides a non-limiting example of a diabetes-management system 10, which allows individuals to actively monitor and record measurements of their blood glucose concentration. As shown in FIG. 1, the diabetes-management system 100 includes a blood-glucose meter (BGM) 110 and a computing device 120. A connection 140 allows the meter 110 to communicate with the computing device 120. The meter 110 obtains point-in-time measurements of blood-glucose concentrations in blood samples and communicates the measurement data to the computing device 120. The computing device 120 executes data management software 126 (executable program instructions stored on computer-readable media) to process the measurement data from the meter 110.

As illustrated in FIG. 1, the meter 110 engages a test sensor 130, which receives a blood sample for analysis. For example, a user may employ a lancing device to pierce a finger or other area of the body to produce a blood sample at the skin surface. The user may then collect the blood sample by placing the test sensor 130 into contact with the sample. As is well known in the art, the test sensor may be an electrochemical test sensor or an optical test sensor.

The meter 110 includes a reaction-detection system for measuring the glucose concentration of the blood sample collected by the test sensor 130. For example, the reaction-detection system may include contacts for the electrodes to detect the electrochemical reaction for an electrochemical test sensor. Alternatively, the reaction-detection system may include an optical detector to detect the chromatic reaction for an optical test sensor. To calculate the actual concentration of analyte from the electrochemical or chromatic reaction measured by the reaction-detection system and to generally control the procedure for testing the sample, the meter 110 employs at least one processor 112, which executes programmed instructions according to a measurement algorithm. Data processed by the processor 112 is stored in a memory 113. Furthermore, the meter 110 includes a user interface 115 that includes a display that shows information regarding the test results.

The computing device 120 may be selected from a variety of processing devices, such as desktop or laptop personal computers (PCs), handheld or pocket personal computers (HPCs), compatible personal digital assistants (PDAs), and smart phones. The processing devices may employ a variety of operating systems and configurations. For example, if the computing device 120 is a desktop or laptop personal computer, the operating system may be a version of Microsoft® Windows® or Apple® Mac® OS. Alternatively, if the computing device 120 is a smart phone, the operating system may correspond with Blackberry® devices from Research in Motion Limited or iPhone® from Apple®.

The computing device 120 includes a processor 122 that is capable of receiving and executing any number of programmed instructions provided on computer-readable media. In addition, the computing device 120 includes a user interface 125 for displaying graphics, text, and/or other audiovisual content. The user interface 125 may be incorporated into the housing of the computing device 120, but it is understood that the user interface 125 may be a separate component, such as a display monitor, that is coupled to the computing device 120.

Although the meter 110 stores test results and provides a user interface 115 to display test results, the test results collected by the meter 110 are communicated to the computing device 120 for additional processing by the data management software 126 and display by the user interface 125. The software 126 on the computing device 120 provides more advanced functionality for managing, processing, and displaying test results and related information. In addition, the computing device 120 provides an enhanced user interface 125 that provides advanced visual and/or audio presentation capabilities. In addition to providing high resolution graphics, the computing device 120 may also allow information to be communicated to the user via audio signals. Moreover, the computing device 120, through network connectivity, may provide the diagnostic system 10 with access to other functionality and data sources.

In general, the computing device 120 may provide processing and presentation capabilities that are not available with the meter 110. It is noted, however, that the meter 110 can fully operate to measure and display an analyte concentration when it is not connected to the computing device 120.

As shown in FIG. 1, the meter 110 includes a communications interface element 111 that enables the meter 110 to connect with the communications interface element 121 of the computing device 120. The communications interface elements 111 and 121 employ wired or wireless interface technologies, such as USB or Bluetooth® technology, to make the devices compatible and enable the appropriate data connections.

As shown further in FIG. 1, the meter 110 includes a power supply 114. The power supply 114 may be a lithium-ion rechargeable battery that receives recharging power from the computing device 120 via the connection 140. In this embodiment, the connection 140 between the meter 110 and the computing device 120 includes signal line connections as well as DC power line connections that allow the meter 110 to draw low voltage current from the computing device 120.

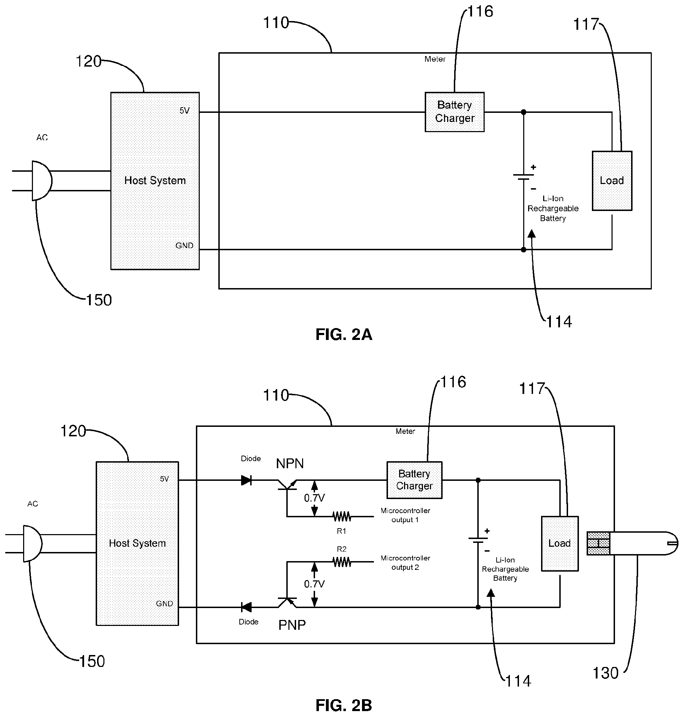

As such, some embodiments employing power line connections between the meter 110 and the computer device 120 protect the user against the danger of electric shock from the power source of the computing device 120. These embodiments provide such protection particularly when the user conducts a test while the meter 110 remains physically connected to the computing device 120. As shown in FIG. 2A, the computing device 120 can draw power from an AC source 150. The computing device 120 is normally isolated from the AC power lines to protect the user. This feature is called primary isolation and protection. However, if the primary isolation/protection for the computing device 120 fails, electricity from the AC power lines can be unsafely delivered via the computing device 120 to the user. Thus, when the meter 110 is plugged into the computing device 120 and the user performs a test with the test strip 130, the user may be shocked when touching the tip of the test strip 130. As a secondary precaution, testing can be disabled when the meter 110 is connected to the computing device 120. However, it is inconvenient for the user to disconnect the meter 110 from the computing device 120 each time to perform a test and to reconnect the meter 110 to transfer test results to the computing device 120. To eliminate this inconvenience, aspects of the present invention electrically isolate the meter 110 from the computing device 120 but allow the meter 110 to remain mechanically connected to the computing device 120 during testing.

In some embodiments, the meter 110 is electrically isolated from power of the computing device 120 through switches and other standard isolation techniques, such as an isolated DC-DC converter. Isolated DC-DC converters normally use a small transformer, called a flyback transformer. Advantageously, isolated DC-DC converters allow the user to conduct testing with the meter 110 while it remains mechanically connected to the computing device 120.

FIGS. 2B-C illustrate example connections between the meter 110 and the computing device 120 that electrically isolate the meter 110 from the power of the computing device 120, particularly when the primary isolation/protection for the computing device fails.

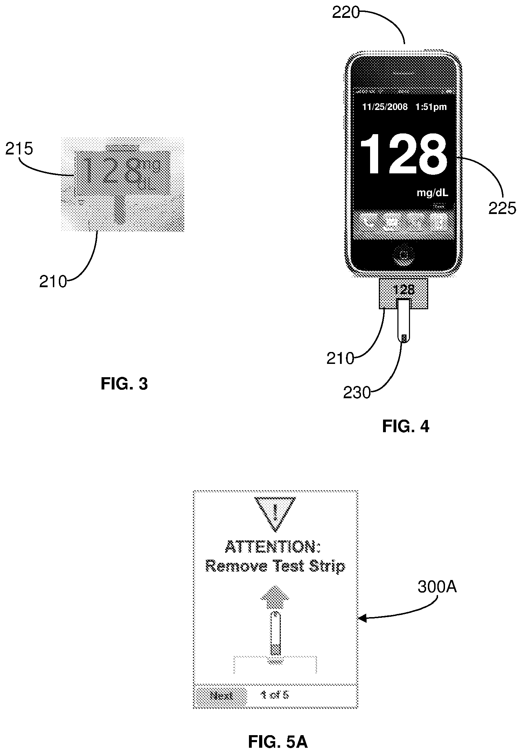

Referring to FIG. 2B, two transistors NPN and PNP during normal operation are turned on when particular voltages are supplied by the microcontroller on the meter 110 to the base input of the transistors NPN and PNP. For example, when the voltage between the base and emitter of the NPN transistor is over 0.7 V, the NPN transistor is turned on. When the voltage between the base and emitter is over -0.7 V, the PNP transistor is turned on. The corresponding two diodes shown in FIG. 4 are forwardly biased so that the current can flow through the diodes when the transistors are turned on. The rechargeable battery 114 inside the meter 110 can then be charged via the battery charger 116. The microcontroller and other electronics inside the meter 110 are shown collectively as load 117 in FIG. 4 because they draw power from the battery 114.

Referring still to FIG. 2B, when the test strip 130 is received by the meter 110, the microcontroller turns off the transistors, and the meter 110 operates solely on power from the battery 114. When the primary isolation/protection breaks down, the two power lines (labeled as 5V and GND) can carry the power line voltages. Therefore, the voltage relative to the meter 110 can be as high as 310 peak volts. The voltage can be positive and negative. When the high voltage is negative, the diodes are inversely biased and do not conduct current. When the high voltage is positive, the NPN and PNP transistors are turned off and do not conduct current. (Diodes or transistors alone will not provide sufficient protection when the polarity of the high voltage changes, as in the case of an AC power lines.) The diodes and the transistors need to have a break down voltage of over 400 V to achieve adequate protection for the user. The values for resistors R1 and R2 shown in FIG. 2B should be sufficiently large. Their values are determined together with the hFE (amplification) of the transistors to provide sufficient protection to the user during normal operation and in the event of a break down of the primary isolation/protection.

Compared to other isolation/protection techniques, such as an isolated DC-DC converter, the embodiment of FIG. 2B can advantageously be implemented at a lower cost and with a smaller geometry, i.e., requiring less board space on the meter 110. The secondary isolation/protection does not necessarily have to be designed with the same level of protection as the primary isolation/protection thus minimizing cost is possible.

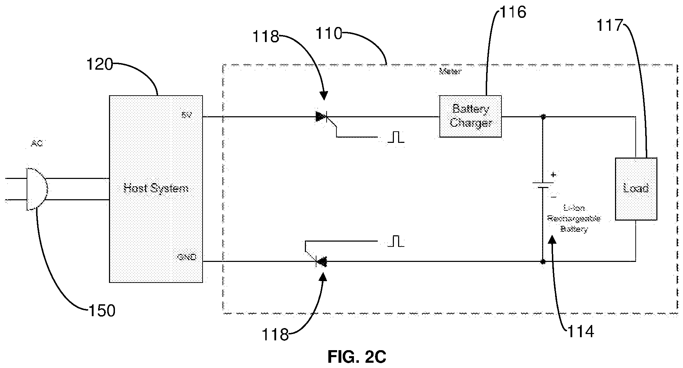

Referring to FIG. 2C, thyristors 118, such as silicon controlled rectifiers (SCRs), are employed for secondary isolation/protection. When the proper activation signal (short pulse) is applied to the gate input, the SCR 118 turns on. There is no need to turn the SCR 118 off, because when there is no AC line failure, the charge current maintains the on-state of the SCR 118. In the event of AC line failure, the AC changes polarity every 1/50 (Europe) or 1/60 seconds (North America) and the SCR turns off when the polarity is reversed thereby protecting the user.

In sum, aspects of the present invention automatically electrically isolate the meter 110 from the power source of a coupled computing device 120 when the user begins to conduct a test with the meter 110, e.g., when the user inserts the test strip 130 into the meter 110. This feature protects the user from failure of the primary isolation/protection for the computing device 120.

As described previously, the computing device 120 may be selected from a variety of processing devices, including portable computing devices. FIG. 3 illustrates a highly portable miniature meter 210 that is compatible with portable computing devices. The size of the miniature meter 210 allows it to be easily coupled to a portable computing device, such as a PALM® handheld, a Blackberry® device, or an Apple® iPhone® device, via a physical connection. For example, the miniature meter 210 may be approximately 20 mm x 15 mm x 5 mm in size. The miniature meter 210 includes a user interface 215, which, for example, may employ graphic liquid crystal display (LCD) or organic light-emitting diode (OLED), segment LCD or OLED, or the like. The graphical user interface 215 may have an area up to approximately 40% of a face of the miniature meter 210 and may have a thickness of approximately 0.3 mm.

As shown in FIG. 4, the miniature meter 210 measures the analyte concentration of the sample on a test sensor. The miniature meter 210 stores the analyte concentration and the analyte concentration can be displayed on the user interface 215. For example, the miniature meter 210 stores a minimum of one week of test results. This memory requirement is lower than other meters. For instance, at four tests per day, there are 28 test results for a week. Each test result requires approximately 8 bytes of storage so the total memory required would be approximately 224 bytes.

As further illustrated in FIG. 4, the miniature meter 210 is coupled to an Apple® iPhone® device 220. The Apple® iPhone® device 220 provides more advanced functionality for managing, processing, and displaying test results and related information. In particular, the Apple® iPhone® device 220 includes a user interface 225 that displays information based on the data received from the miniature meter 210.

Generally, the computing device 120 executes data management software 126 and presents data and information relating to the meter 110 on the user interface 125 of the computing device 110. Moreover, the computing device 120 can present the data and information while the meter 110 remains coupled to the computing device during testing. In particular, the user interface 125 presents clear and easy-to-follow instructions for conducting the testing procedure on the meter 110. For example, the computing device 120 executes software 126 that is stored on computer-readable media to present illustrative graphics, textual information, and/or audio for each step during the testing procedure. As such, the user receives clear step-by-step instructions to minimize the chance of user error during the testing procedure. In some embodiments, the software 126 may enhance the presentation of instructions by employing animation. For example, the user interface 125 may show an animated person or character (e.g., a cartoon depiction of a health care provider, diabetes care educator, the user, or a person of the user's choice) to guide the user through the steps in a more engaging and personable manner.

In some embodiments, the user interface 125 also shows illustrative graphics, textual information, and/or audio that guide or assist the user through appropriate steps when an error or exceptional condition occurs during the testing procedure. For example, the temperature of the reagent on the test sensor 130 may affect the accuracy of the concentration of analyte calculated by the meter, as the level of reaction between the analyte and the reagent may be dependent on the temperature of the reagent. As such, some embodiments of the present invention determine a temperature for the reagent and use this calculated temperature to produce a more accurate measurement of the analyte concentration. In particular, the meter 110 has a temperature-measuring system which provides a calculated temperature as a variable input for a measurement algorithm. Although the actual measurement is corrected based on the actual test sensor temperature, in some cases, however, the accuracy of the testing procedure is improved by replacing the test sensor 130 with one that has a temperature within a preferred range, e.g., closer to the ambient temperature. Thus, when the temperature-measuring system determines that the temperature of the test sensor 130 is not in an acceptable range, the user interface may present illustrative graphics and textual information (as well as audio) that instruct the user to replace the test sensor when the diagnostic system senses that the test sensor temperature is outside a preferred range. Examples of such illustrative graphics and textual information are shown in FIGS. 5A-E.

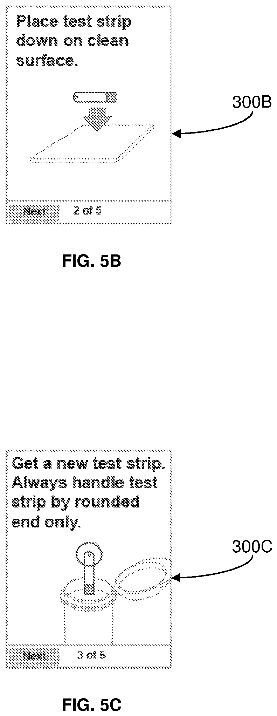

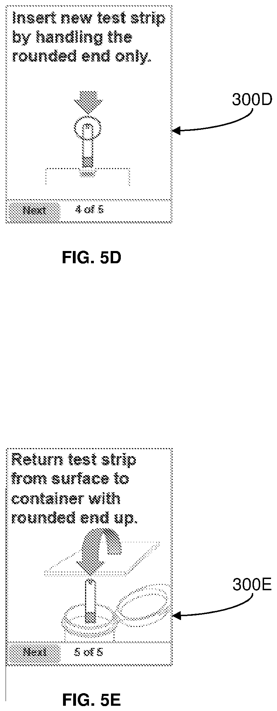

The illustrative graphics and textual information on the screen 300A shown in FIG. 5A alerts the user to an exceptional condition regarding the test sensor 130 and instructs the user to remove the test sensor 130. The screen 300B in FIG. 5B then instructs the user to place the removed test sensor 130 on a clean surface. As shown in FIG. 5C, the screen 300C instructs the user to retrieve another test sensor from a test sensor container, reminding the user how to handle the test sensors 130. Because the removed test sensor 130 has been placed on the clean surface, the user cannot accidentally select the removed test sensor 130 from the container. The screen 300D in FIG. 5D then instructs the user to insert the new test sensor into the meter 110, reminding the user again how to handle the test sensors 130. As shown in FIG. 5E, the screen 300E finally instructs the user to return the removed test sensor 130 to the test sensor container for later use.

The user may step through the sequence of screens 300A-E corresponding to FIGS. 5A-E by operating the "Next" pushbutton when the user is ready to move to the subsequent screen. Alternatively, the user interface 125 may show an automated slideshow that loops through screens 300A-E. Alternatively, as discussed previously, the graphical information in screens 300A-E may be shown as an animated presentation.

While it may be advantageous to present the illustrative graphics, textual information, and/or audio during the actual testing procedure or operation of the meter 110, it is understood that the illustrative graphics, textual information, and/or audio may be shown separately as a tutorial. Furthermore, it is understood that the user interface 125 may provide any information that may guide or assist the user in the operation of the meter and is not limited to presenting the types of information shown in screens 300A-E. For example, the user interface 125 may present screens that guide a user through a migration from one type of meter to another; such a feature would promote loyalty to a particular brand or line of meters.

As described previously, the computing device 120 includes data management software 126. The software 126 on the computing device 120 includes a collection of programs or computer code that receives and processes data measured by the meter 110. The software 126 processes and/or displays this input in a manner that is desired by the user. This information may be used by, for example, a user, home care provider (HCP), and/or a physician. Advantageously, the software 126 can provide the advanced displays and data processing that may be required by a user who tests multiple times a day (e.g., about six to about ten times a day). For example, the software 126 may include a product similar to WINGLUCOFACTS® Diabetes Management Software available from Bayer HealthCare LLC (Tarrytown, New York). As such, the software 126 may provide a complete tool kit that receives and stores test results from a blood-glucose measurement system, receives and stores other testing information such as test times and meal markers, tracks test results in an electronic logbook, calculates averages and provides statistical analysis of outlier test results, summarizes and provides feedback on the test results, provides a customizable graphical user interface (GUI), displays user-friendly charts and graphs of the test results, tracks test results against user-specific target ranges, provides predictive analysis, and/or sends data to healthcare professionals via fax, email, etc.

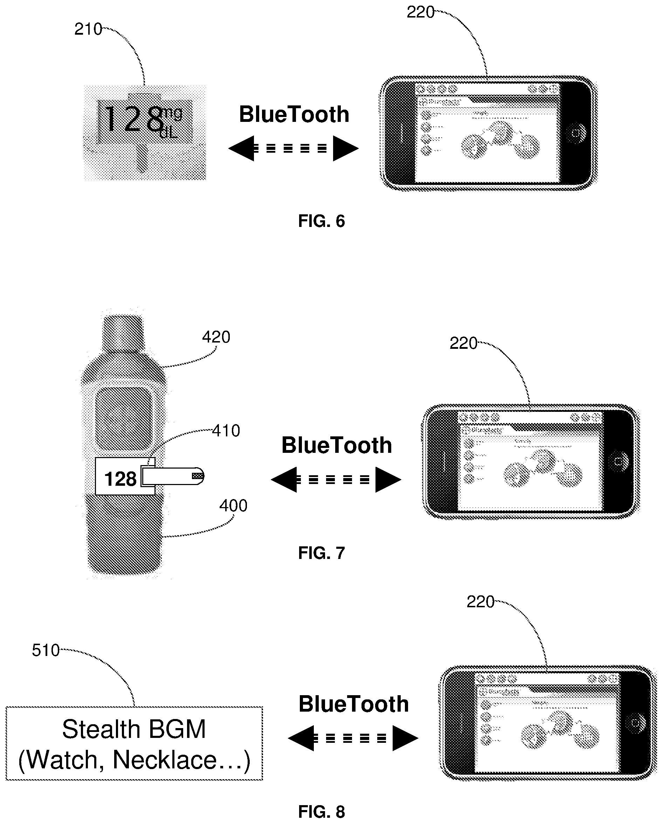

Diagnostic systems according to the aspects of the present invention employ a variety of architectures and configurations. As described previously with reference to FIG. 4, a meter is configured as a miniature meter 210 that is highly portable and that can be coupled to a portable computing device, such as the Apple® iPhone® 220. In an alternative embodiment, however FIG. 6 illustrates the miniature meter 210 communicating wirelessly, e.g., via Bluetooth®, with the Apple® iPhone® 220. As shown in FIG. 5, the Apple® iPhone® 220 is executing data management software.

In another alternative embodiment, FIG. 7 illustrates a meter that is configured as a miniature meter component 410 of an integrated lancet device 400. The integrated lancet device 400 combines a lancet 420 for producing a sample at a skin surface with a meter 410 for analyzing the sample. The meter 410 may be integral with the lancet 420 or may be removably coupled to the lancet 420. By way of example, the meter 410 in FIG. 7 communicates wirelessly, e.g., via Bluetooth®, with the Apple® iPhone® 220. However, the communication may be via a wired connection.

In yet another embodiment, FIG. 8 illustrates a meter that is configured as a "stealth" meter 510 that is discretely configured as a watch, necklace, or the like. By way of example, the "stealth" meter 510 in FIG. 8 communicates wirelessly, e.g., via Bluetooth®, with the Apple® iPhone® 220. However, the communication may be via a wired connection.

It is understood that the meter 110, rather than the computing device 120, may be employed to execute its own software and present information, such as that shown in screens 300A-E of FIGS. 5A-E. This is particularly advantageous in embodiments that do not allow any communication between the meter 110 and the computing device 120 when testing is being conducted with the meter.

Furthermore, it is also understood that aspects of the present invention are not limited to blood-glucose measurement systems and are applicable to broader diagnostic systems. Analytes that may be analyzed include glucose, lipid profiles (e.g., cholesterol, triglycerides, LDL and HDL), microalbumin, hemoglobin A1_{C} fructose, lactate, or bilirubin. It is contemplated that other analyte information may be determined (e.g., analyte concentrations). The analytes may be in, for example, a whole blood sample, a blood serum sample, a blood plasma sample, other body fluids like ISF (interstitial fluid) and urine, and non-body fluids.

While the invention is susceptible to various modifications and alternative forms, specific embodiments and methods thereof have been shown by way of example in the drawings and are described in detail herein. It should be understood, however, that it is not intended to limit the invention to the particular forms or methods disclosed, but, to the contrary, the intention is to cover all modifications, equivalents and alternatives falling within the spirit and scope of the invention.

## Claims

1. A system (10) for determining an analyte concentration in a fluid sample, the system (10) comprising:
a meter (110) that is configured to receive a test sensor (130) during a testing procedure, the test sensor (130) receiving a fluid sample during the testing procedure, the meter (110) including a measurement system (112, 113) that determines a measurement of a concentration of an analyte in the fluid sample, the meter (110) including a rechargeable battery (114) and a connection (140), the connection (140) being configured to electrically and mechanically couple the meter (110) to external computing devices; and
a computing device (120) electrically and mechanically coupled to the meter (110) via the connection (140), the computing device (120) receiving and processing the measurement from the meter (110), the computing device (120) including a power source (150), **characterized in that** the rechargeable battery (114) of the meter (110) is rechargeable with power from the power source (150), wherein the meter (110) receives the power via the connection (140),wherein the connection (140) is configured to electrically isolate the meter (110) from the power source of the computing device (120) when the testing procedure is initiated while the meter (110) remains mechanically coupled to the computing device (120) via the connection (140).

2. The system (10) of claim 1, wherein the testing procedure is initiated upon the meter (110) receiving the test sensor (130).

3. The system (10) of claim 1, wherein the connection (140) includes an isolated DC-DC converter.

4. The system (10) of claim 1, wherein the meter (110) includes a microcontroller and the connection (140) includes a transistor and a diode, the microcontroller turning the transistor on and current flowing through the diode from the computing device (120) when the test sensor (130) is not received by the meter (110), and the microcontroller turning the transistor off and the meter (110) operating solely on power from the battery when the test sensor (130) is received by the meter (110).

5. The system (10) of claim 5, wherein the transistor and the diode operate to prevent the flow of current from the computing device (120) when a high voltage occurs over the connection due to a failure on the computing device (120).

6. The system (10) of claim 1, wherein the connection (140) includes a silicon controlled rectifier (SCR), the SCR turning off when a failure occurs on the computing device (120) and a reversal of polarity occurs at the SCR with a resulting flow of alternating current.

7. The system (10) of claim 1, wherein the connection (140) includes signal line connections and DC power line connections configured to transmit low voltage current from the computing device (120) to the meter (110).

8. The system (10) of claim 1, wherein the connection (140) includes first and second transistors with first and second diodes forwardly biased such that current flows through the diodes when the transistors are turned on.

9. The system (10) of claim 8, wherein the meter (110) includes a microcontroller connected to the first and second transistors, and wherein the transistors are turned on when the microcontroller supplies minimum voltages to the base inputs of the transistors.

10. The system (10) of claim 8, wherein the connection (140) further includes first and second resistors connected to the first and second transistors, respectively.

## Patentansprüche

1. System (10) zum Bestimmen einer Analytkonzentration in einer Fluidprobe, wobei das System (10) umfasst:
ein Messgerät (110), das konfiguriert ist, um während eines Testvorgangs einen Testsensor (130) aufzunehmen, wobei der Testsensor (130) während des Testvorgangs eine Fluidprobe erhält, wobei das Messgerät (110) ein Messsystem (112,113) umfasst, das einen Messwert einer Konzentration eines Analyts in der Fluidprobe bestimmt, wobei das Messgerät (110) eine wiederaufladbare Batterie (114) und eine Verbindung (140) umfasst, wobei die Verbindung (140) konfiguriert ist, das Messgerät (110) elektrisch und mechanisch mit externen Computervorrichtungen zu verbinden; und
eine Computervorrichtung (120), die mit dem Messgerät (110) elektrisch und mechanisch über die Verbindung (140) verbunden ist, wobei die Computervorrichtung (120) den Messwert von dem Messgerät (110) empfängt und verarbeitet, wobei die Computervorrichtung (120) eine Energiequelle (150) umfasst, **dadurch gekennzeichnet, dass**
die wiederaufladbare Batterie (114) des Messgeräts (110) mit Energie von der Energiequelle (150) wiederaufladbar ist, wobei das Messgerät (110) die Energie über die Verbindung (140) empfängt, wobei die Verbindung (140) konfiguriert ist, dass Messgerät (110) von der Stromquelle der Computervorrichtung (120) elektrisch zu isolieren, wenn der Testvorgang initiiert wird, während das Messgerät (110) mechanisch über die Verbindung (140) mit der Computervorrichtung (120) verbunden bleibt.

2. System (10) nach Anspruch 1, wobei der Testvorgang initiiert wird, wenn das Messgerät (110) den Testsensor (130) aufnimmt.

3. System (10) nach Anspruch 1, wobei die Verbindung (140) einen isolierten DC-DC-Wandler umfasst.

4. System (10) nach Anspruch 1, wobei das Messgerät (110) einen Mikrocontroller umfasst und die Verbindung (140) einen Transistor und eine Diode umfasst, wobei der Mikrocontroller den Transistor einschaltet und Strom von der Rechenvorrichtung durch die Diode fließt (120), wenn der Testsensor (130) nicht von dem Messgerät (110) aufgenommen ist, und der Mikrocontroller den Transistor ausschaltet und das Messgerät (110) ausschließlich mit Strom von der Batterie arbeitet, wenn der Testsensor (130) von dem Messgerät (110) aufgenommen ist.

5. System (10) nach Anspruch 4, wobei der Transistor und die Diode so betrieben werden, dass sie den Stromfluss von der Rechenvorrichtung (120) verhindern, wenn, aufgrund eines Fehlers auf der Rechenvorrichtung (120), eine hohe Spannung auf der Verbindung auftritt.

6. System (10) nach Anspruch 1, wobei die Verbindung (140) einen siliziumgesteuerten Gleichrichter (SCR) umfasst, wobei der SCR abgeschaltet wird, wenn ein Fehler an der Rechenvorrichtung (120) auftritt und eine Umkehr der Polarität an der SCR mit einem resultierenden Fluss von Wechselstrom auftritt.

7. System (10) nach Anspruch 1, wobei die Verbindung (140) Signalleitungsverbindungen und DC Leistungsleitungsverbindungen umfasst, die konfiguriert sind, um Niederspannungsstrom von der Computervorrichtung (120) zu dem Messgerät (110) zu übertragen.

8. System (10) nach Anspruch 1, wobei die Verbindung (140) einen ersten und einen zweiten Transistor mit ersten und zweiten Dioden umfasst, die in Durchlassrichtung derart vorgespannt sind, dass Strom durch die Dioden fließt, wenn die Transistoren eingeschaltet sind.

9. System (10) nach Anspruch 8, wobei das Messgerät (110) einen Mikrocontroller aufweist, der mit dem ersten und dem zweiten Transistor verbunden ist, und wobei die Transistoren eingeschaltet sind, wenn der Mikrocontroller an die Basiseingänge der Transistoren minimale Spannungen anlegt.

10. System (10) nach Anspruch 8, wobei die Verbindung (140) ferner erste und zweite Widerstände umfasst, die mit dem ersten beziehungsweise dem zweiten Transistor verbunden sind.

## Revendications

1. Système (10) pour déterminer une concentration d'analyte dans un échantillon de fluide, le système (10) comprenant :
un compteur (110) qui est configuré pour recevoir un capteur de test (130) au cours d'une procédure de test, le capteur de test (130) recevant un échantillon de fluide au cours de la procédure de test, le compteur (110) comprenant un système de mesure (112, 113) qui détermine une mesure d'une concentration d'un analyte dans l'échantillon de fluide, le compteur (110) comprenant une batterie rechargeable (114) et une connexion (140), la connexion (140) étant configurée pour coupler électriquement et mécaniquement le compteur (110) à des dispositifs extérieurs de calcul ; et
un dispositif de calcul (120) électriquement et mécaniquement couplé au compteur (110) via la connexion (140), le dispositif de calcul (120) recevant et traitant la mesure provenant du compteur (110), le dispositif de calcul (120) comprenant une source d'alimentation en courant électrique (150),
**caractérisé en ce que** la batterie rechargeable (114) du compteur (110) est rechargeable avec du courant électrique provenant de la source d'alimentation en courant électrique (150), où le compteur (110) reçoit l'alimentation en courant électrique via la connexion (140), où la connexion (140) est configurée pour isoler électriquement le compteur (110), de la source d'alimentation en courant électrique du dispositif de calcul (120) quand la procédure de test est déclenchée, tandis que le compteur (110) reste mécaniquement couplé au dispositif de calcul (120) via la connexion (140).

2. Système (10) selon la revendication 1, dans lequel la procédure de test est déclenchée au moment où le compteur (110) reçoit le capteur de test (130).

3. Système (10) selon la revendication 1, dans lequel la connexion (140) comprend un convertisseur CC / CC isolé.

4. Système (10) selon la revendication 1, dans lequel le compteur (110) comprend un microcontrôleur, et la connexion (140) comprend un transistor et une diode, le microcontrôleur met en marche le transistor et du courant traverse la diode depuis le dispositif de calcul (120) quand le capteur de test (130) n'est pas reçu par le compteur (110), et le microcontrôleur arrête le transistor et le compteur (110) fonctionne seulement sur l'alimentation en courant électrique de la batterie quand le capteur de test (130) est reçu par le compteur (110).

5. Système (10) selon la revendication 4, dans lequel le transistor et la diode fonctionnent pour empêcher de passer le flux de courant provenant du dispositif de calcul (120) quand une haute tension se produit sur la connexion en raison d'une panne sur le dispositif de calcul (120).

6. Système (10) selon la revendication 1, dans lequel la connexion (140) comprend un redresseur commandé au silicium (SCR), le redresseur SCR se mettant hors circuit quand une panne se produit sur le dispositif de calcul (120), et une inversion de polarité se produit au niveau du redresseur SCR, avec un flux de courant alternatif qui en résulte.

7. Système (10) selon la revendication 1, dans lequel la connexion (140) comprend des connexions de lignes de signaux et des connexions de lignes d'alimentation en courant continu, lesdites connexions étant configurées pour transmettre du courant à basse tension depuis le dispositif de calcul (120) jusqu'au compteur (110).

8. Système (10) selon la revendication 1, dans lequel la connexion (140) comprend des premiers et des deuxièmes transistors ayant des premières et des deuxièmes diodes polarisées en sens direct, de manière telle que du courant traverse les diodes quand les transistors sont mis en marche.

9. Système (10) selon la revendication 8, dans lequel le compteur (110) comprend un microcontrôleur connecté aux premiers et aux deuxièmes transistors, et dans lequel les transistors sont mis en marche quand le microcontrôleur fournit des tensions minimales aux entrées de base des transistors.

10. Système (10) selon la revendication 8, dans lequel la connexion (140) comprend en outre des premières et des deuxièmes résistances connectées respectivement aux premiers et aux deuxièmes transistors.
